# EUROPÄISCHE PATENTSCHRIFT

(11) **EP 1 216 665 B1**
(45) Veröffentlichungstag und Bekanntmachung des Hinweises auf die Patenterteilung: **06.02.2008**
(21) Anmeldenummer: 01250443.7
(22) Anmeldetag: 17.12.2001
(51) Int. Cl.: A61F 2/82, A61B 19/00

(54) **Verfahren zum Anbringen eines Markerelements an einem Implantat**
Method for joining a marker element to an implant
Procédé pour adjoindre un élément marqueur à un implant

(30) Priorität: 18.12.2000 DE 10064596
(43) Veröffentlichungstag der Anmeldung: 26.06.2002
(73) Patentinhaber: BIOTRONIK GmbH & Co. KG, 12359 Berlin (DE)
(72) Erfinder: Schaldach, Max, verstorben (DE); Gobrecht, Jens, 5412 Gebenstorf (CH); Schmitz, Klaus-Peter, 18055 Rostock (DE); Rzany, Alexander, 90449 Nürnberg (DE); Behrend, Detlef, 18119 Rostock (DE)
(74) Vertreter: Lindner-Vogt, Karin L.

(56) Entgegenhaltungen:
- EP-A- 0 894 503
- WO-A-97/33534
- WO-A-99/15108
- US-A- 5 632 771

## Beschreibung

Die vorliegende Erfindung betrifft ein Verfahren zum Anbringen eines Markerelements an einem zum Implantieren in den menschlichen oder tierischen Körper vorgesehenen Implantat, insbesondere einem Stent, mit einem Grundkörper und einer in diesem Grundkörper vorgesehenen Ausnehmung zur Aufnahme des Markerelements. Weiterhin betrifft sie ein Implantat, insbesondere einen Stent, welches mit einem solchen Markerelement versehen ist.

Eine Vielzahl derzeit gebräuchlicher Implantate werden in den menschlichen oder tierischen Körper beispielsweise unter Zuhilfenahme von Katheter oder dergleichen eingesetzt, ohne dass der Chirurg die Implantationsstelle direkt einsehen kann.

Häufig ist es daher erforderlich, dass die korrekte Position des Implantats mit anderen Mitteln nachvollzogen wird. Hierzu werden meist bildgebende, beispielsweise mit Röntgenstrahlen arbeitende Geräte eingesetzt, welche es dem Chirurgen erlauben, anhand der erhaltenen Abbildungen die Positionierung des Implantats zu beurteilen.

Da viele Implantatwerkstoffe keinen ausreichend kontrastreichen Bildeindruck auf den mit den derzeit gebräuchlichen Methoden gewonnenen Abbildungen hinterlassen, werden in der Regel so genannte Marker an definierten Positionen am Implantat angebracht. Der Werkstoff dieser Marker desto gewählt, dass er einen ausreichend kontrastreichen Bildeindruck ergibt, welcher Rückschlüsse auf die Position des Implantats zulässt.

Derartige Marker bestehen in der Regel aus Festkörpern, welche für die zur Bildgebung verwendete Strahlung undurchlässig sind. Ein solcher Festkörper ist in die Ausnehmung im Grundkörper des Implantats eingesetzt und mit dem Grundkörper durch Verpressen und zusätzlich oder alternativ durch Verschweißen verbunden.

So ist beispielsweise aus der WO 97/33534 ein Stent bekannt, bei dem einzelne röntgenopake Markerelemente aus Gold in Ausnehmungen im Grundkörper des Stents eingesetzt und mit diesem verpresst sind, um die mechanische Verbindung zwischen Markerelement und Stentgrundkörper herzustellen. Hierbei werden die Markerelemente zum Teil auch nach Art einer Niete verformt. Ähnliche Röntgenmarker für einen Stent sind aus der US 6,022,374 bekannt. Diese weisen gegenüber der Ausnehmung ein Übermaß auf und werden unter Verformung des die Ausnehmung begrenzenden Stentmaterials in die Ausnehmung eingepresst.

Beide Varianten weisen den Nachteil auf, dass das Implantat im Bereich der Ausnehmung beim Verpressen teils recht hohen mechanischen Belastungen unterworfen ist. Gerade der relativ fragilen Gebilden, wie sie beispielsweise Stents darstellen, kann es in diesem Bereich schnell zu einer unerwünschten Beschädigung des Grundkörpers kommen. Diese ist besonders schädlich, wenn sie nicht sofort erkennbar ist und es gegebenenfalls erst im implantierten Zustand zum Versagen kommt. So kann es beispielsweise bei einen Stent zu unerwünschten scharfen Bruchkanten kommen, welche möglicherweise sogar das umliegende Gewebe schädigen. Ebenso kann es natürlich auch zu einem unerwünschten Lösen das Markerelements vom Implantat kommen.

Bei anderen bekannten Varianten wird das Markerelement mit dem angrenzenden Grundkörper des Implantats verschweißt. Dies ist beispielsweise für einen Stent aus der US 5,632,771 bekannt. Hier besteht, wie im Übrigen auch bei den obengenannten Varianten, der Nachteil, dass die Ausnehmung und das Markerelementmit einer relativ genauen Passung hergestellt werden müssen, um sicherzustellen, dass ein ausreichender mechanischer Verbund erzielt wird. Es ergibt sich somit für alle diese Varianten eine relativ aufwändige Herstellung. Zudem ist bei allen bekannten Varianten der Spielraum bei der Variation der Geometrie des Markers bzw. der Ausnehmung unter wirtschaftlichen Gesichtspunkten eingeschränkt, da für unterschiedliche Ausnehmungen jeweils unterschiedliche Marker hergestellt werden müssen.

Der vorliegenden Erfindung liegt da die Aufgabe zu Grunde, ein gattungsgemäßes Verfahren zum Anbringen eines Markerelements an einem Implantat sowie ein gattungsgemäßes Implantat zur Verfügung zu stellen, die die obengenannten Nachteile nicht oder zumindest in geringerem Maße aufweisen und insbesondere eine einfache und flexible Herstellung gewährleisten.

Diese Aufgabe wird ausgehend von einem Verfahren gemäß dem Oberbegriff des Anspruchs 1 durch die im kennzeichnenden Teil des Anspruchs 1 angegebenen Merkmale gelöst. Ebenso wird sie ausgehend von einem Implantat gemäß dem Oberbegriff des Anspruchs 12 durch die im kennzeichnenden Teil des Anspruchs 12 angegebenen Merkmale gelöst.

Der vorliegenden Erfindung liegt die technische Lehre zu Grunde, dass man eine besonders einfache und flexible Herstellung eines Implantats mit einem in einer Ausnehmung des Grundkörpers des Implantats angeordneten Markerelement erzielt, wenn zur Ausbildung wenigstens eines Teils des Markerelements ein verfestigbares Material oder Materialgemisch in die Ausnehmung eingebracht und dort verfestigt wird.

Bei dem eingebrachten Material oder Materialgemisch handelt es sich bevorzugt um ein fließ- oder schüttfähiges Material oder Materialgemisch. Durch die Fließ- bzw. Schüttfähigkeit des verwendeten verfestigbaren Materials bzw. Materialgemisches ist zum einen sichergestellt, dass sich das entstehende Markerelement ohne weiteres an beliebige Geometrieen der Ausnehmung anpasst. Dies hat zum einen der Folge, dass eine besonders hohe Flexibilität bei der Gestaltung der Ausnehmungen besteht und zum anderen bei deren Fertigung keine besonderen Maßtoleranzen oder dergleichen einzuhalten sind. Hierdurch erleichtert sich die Fertigung des Implantats erheblich.

Ein weiterer Vorteil der erfindungsgemäßen Lösung liegt darin, dass beim Anbringen des Markerelements keine übermäßig hohen Spannungen zwischen dem Markerelement und dem angrenzenden Grundkörper des Implantats auftreten, sodass weder das Markerelement noch der Grundkörper des Implantats übermäßigen mechanischen Belastungen, insbesondere hohen Vorspannungen unterworfen sind. Die Wahrscheinlichkeit, dass es bei der Herstellung oder im Einsatz zu einem Versagen des Markerelements oder des angrenzenden Grundkörpers kommt, ist somit gegenüber den aus dem Stand der Technik bekannten Varianten erheblich reduziert.

Bei dem verwendeten Material bzw. Materialgemisch kann es sich um ein beliebiges ausreichend biokompatibles Material bzw. Materialgemisch handeln, welches ausreichend verfestigbar ist, um ein ausreichend stabiles Markerelement sowie einen ausreichend sicheren Verbund zwischen dem Markerelement und dem angrenzenden Grundkörper herzustellen.

Das verwendete Material bzw. Materialgemisch bildet dabei vorzugsweise das gesamte Markerelement. Hierzu kann es aus einer einzelnen entsprechend verfestigbaren Komponente bestehen, die zudem eine ausreichende Undurchlässigkeit für die zur Bildgebung verwendete Strahlung aufweist.

Die Erfindung besteht darin, dass Partikel oder größere Teile eines entsprechend strahlungsundurchlässigen Materials einem entsprechenden verfestigbaren Material bzw. Materialgemisch beigemischt sind. So ist es beispielsweise denkbar, dass das schüttfähige Material bzw. Materialgemisch aus einem Granulat besteht, dessen Körner aus einem oder mehreren Partikeln eines entsprechend strahlungsundurchlässigen Materials bestehen, die von einem verfestigbaren Material bzw. Materialgemisch umschlossen sind. Ebenso ist es möglich, die Ausnehmung mit einer Suspension aus einem aushärtenden flüssigen Material bzw. Materialgemisch und solchen strahlungsundurchlässigen Partikeln auszugießen. Im Endergebnis können aber auch größere Teile aus einem solchen strahlungsundurchlässigen Material ganz oder teilweise von einem entsprechenden verfestigbaren Material umschlossen sein.

Eine weitere Variante stellen fließfähige oder pastöse, verfestigbare und entsprechend strahlungsundurchlässige Materialien, wie beispielsweise das aus der Zahnheilkunde bekannte Amalgam, dar, welche ohne Energiezufuhr, d. h. kalt aushärten. Es versteht sich, dass natürlich auch diese in Verbindung mit den genannten eingeschlossenen oder umschlossenen Partikeln bzw. Teilen eingesetzt werden können.

Es ist bei allen diesen Varianten lediglich erforderlich, dass ein ausreichend stabiler mechanischer Verbund zwischen dem Grundkörper, dem verfestigbaren Material bzw. Materialgemisch und gegebenenfalls den Teilen aus dem strahlungsundurchlässigen Material erzielt wird. Hierzu können durch eine entsprechende Gestaltung des Grundkörpers im Bereich, der die Ausnehmung begrenzt, oder durch die Verwendung entsprechender Formen etc. beim Verfüllen der Ausnehmung mit dem fließfähigen oder schüttfähigen Material bzw. Materialgemisch Hinterschneidungen zwischen dem Grundkörper und dem sich ergebenden Markerelement erzeugt werden, die eine formschlüssige Verbindung zwischen Grundkörper und Markerelement bewirken. Ebenso ist es aber möglich, dass dieser stabile Verbund zusätzlich oder alternativ auch dadurch erzielt wird, dass das fließ- oder schüttfähige Material oder Materialgemisch während des Verfestigungsvorgangs mit dem Material des Grundkörpers verbunden wird. Dies kann durch Adhäsion oder durch eine Materialverschmelzung, beispielsweise ein Verschweißen, erfolgen. Als Materialien für das verfestigbare Material bzw. Materialgemisch kommen sowohl Kunststoffe bzw. Kunststoffgemische, Metalle bzw. Metalllegierungen sowie Keramiken in Betracht.

Bei bevorzugten der Varianten des erfindungsgemäßen Verfahrens ist vorgesehen, dass das fließ- oder schüttfähige Material oder Materialgemisch ein sinterfähiges Granulat oder Pulver ist, das in der Ausnehmung durch Sintern verfestigt wird, da ein solches Granulat oder Pulver eine besonders einfache Handhabung ermöglichen.

Vorteilhafte Ausgestaltungen des erfindungsgemäßen Verfahrens zeichnen sich dadurch aus, dass der Verfestigungsvorgang einen endothermen Schritt umfasst und wenigstens ein Teil der Prozessenergie in dem endothermen Schritt lokal im Bereich der Ausnehmung eingebracht wird. Hierdurch lässt sich in vorteilhafter Weise eine besonders gezielte und gut steuerbare Verfestigung mit dem Ergebnis sowohl eines stabilen Markerelements als auch einer stabilen Verbindung zwischen dem Markerelement und dem Grundkörper.

Die Prozessenergie kann dabei in beliebiger, auf das zu verfestigende Material bzw. Materialgemische abgestimmter Weise gezielt lokal eingebracht werden. Vorzugsweise wird sie in dem endothermen Schritt durch gezielte Bestrahlung des im Bereich der Ausnehmung angeordneten Materials bzw. Materialgemisches eingebracht. Hierbei kann beispielsweise ein Laser verwendet werden, da sich dieser besonders einfach und gut gezielt einsetzen lässt. Es sind jedoch auch beliebige andere Varianten der Energieeinbringung einsetzbar. So kann beispielsweise ein Pulver oder Granulat zu mechanischen Schwingungen angeregt werden, die dann auf Grund der Reibung zwischen den Partikeln eine Erwärmung bewirken. Eine solche Anregung kann beispielsweise mittels Ultraschall erfolgen. Ebenso können aber auch gezielt entsprechend geformte Heizflächen bzw. Heizelemente oder dergleichen eingesetzt werden. Diese können zusätzlich dazu verwendet werden, Druck auf das zu verfestigende Material bzw. Materialgemisch auszuüben. Solche Stempel etc. zur Druckerzeugung können natürlich aber auch im Zusammenhang mit den anderen Varianten der Energieeinbringung eingesetzt werden.

Bei anderen Varianten wird wenigstens ein Teil der Prozessenergie in dem endothermen Schritt elektrisch durch Erzeugung eines Stromflusses durch das im Bereich der Ausnehmung angeordnete fließ- oder schüttfähige Material oder Materialgemisch eingebracht. Hierbei kann das Material bzw. Materialgemisch so gewählt sein, dass es für den Strom einen großen Widerstand darstellt, mit dem Ergebnis, dass in ihm ein großer Teil der zugeführten Energie in Wärme umgesetzt wird.

Es versteht sich, dass die angeführten Möglichkeiten der Einbringung der Prozessenergie in dem endothermen Schritt auch beliebig miteinander kombiniert werden können.

Die vorliegende Erfindung betrifft weiterhin ein Implantat, insbesondere einen Stent, zum Implantieren in den menschlichen oder tierischen Körper mit einem in der oben beschriebenen Weise hergestellten bzw. angebrachten Markerelement, welcher gleichermaßen die oben beschriebenen Vorteile aufweist.

Bei besonders bevorzugten Varianten des erfindungsgemäßen Implantats sind die Ausnehmung und zusätzlich oder alternativ das Markerelement sowie zusätzlich oder alternativ deren Anordnung bezüglich des Grundkörpers zur Kennzeichnung wenigstens einer Eigenschaft des Implantats ausgebildet. Ebenso ist es aber möglich durch die Gestalt bzw. die Anordnung eines Markerelements oder mehrerer Markerelemente zueinander eine solche Kennzeichnung zu bewerkstelligen. Hierbei kann es beispielsweise um eine Produktkennzeichnung handeln. Ebenso ist es auch möglich, in dieser Weise beliebige andere Informationen mit Implantat zu verknüpfen. So kann sich in der geschilderten Weise z. B. das Herstellungsdatum, das Material etc. des Implantats ergeben.

Ebenso ermöglicht die Erfindung, mit geringem Aufwand Markierungen in Bereichen des Implantats Anbringen, wie von besonderem Interesse sind. So ist es beispielsweise bei einem zum Einsatz im Bereich von Gefäßverzweigungen vorgesehenen so genannten Bifurkationsstent möglich, die seitlichen Öffnungen einfach in der geschilderten Weise zu kennzeichnen.

Weitere bevorzugte Ausführungsformen der vorliegenden Erfindung ergeben sich aus den Unteransprüchen bzw. werden nachstehend unter Bezugnahme auf die beigefügten Zeichnungen beschrieben. Es zeigen:
- Figur 1: einen schematischen Teilschnitt durch eine Anordnung zur Durchführung einer bevorzugten Variante des erfindungsgemäßen Verfahrens;
- Figur 2: eine schematische Schnittansicht des Details II aus Figur 1;
- Figur 3: einen schematischen Teilschnitt durch eine Anordnung zur Durchführung einer weiteren bevorzugten Variante des erfindungsgemäßen Verfahrens;
- Figur 4A bis 4D: schematische Teilschnitte durch bevorzugte Ausführungen des erfindungsgemäßen Implantats;
- Figur 5: einen schematischen Teilschnitt durch eine weitere Ausführung des Implantats, nicht Teil der Erfindung.
- Figur 6: eine schematische Draufsicht auf eine bevorzugte Ausführung eines erfindungsgemäßen Stents.

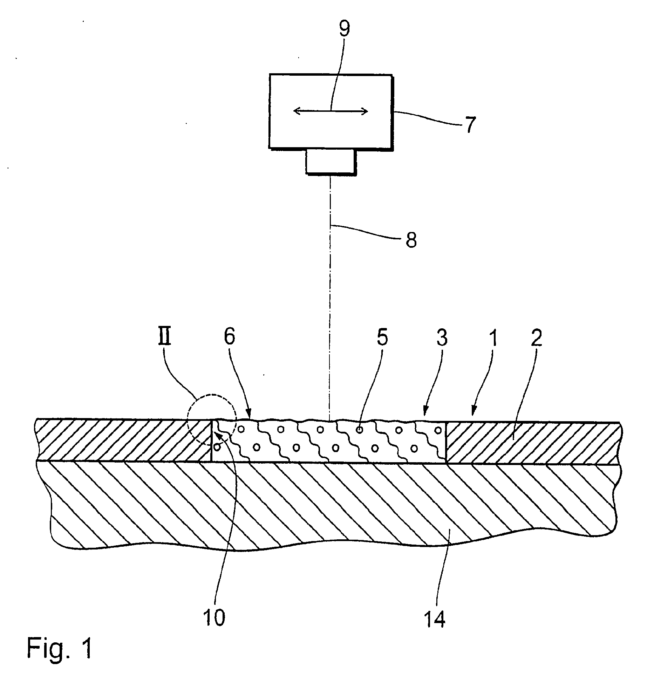

Figur 1 zeigt einen schematischen Teilschnitt durch eine Anordnung zur Durchführung einer bevorzugten Variante des erfindungsgemäßen Verfahrens mit einem Implantat 1, in dessen Grundkörper 2 eine Ausnehmung 4 vorgesehen ist. In diese Ausnehmung 4 wurde ein verfestigbares, schüttfähiges Materialgemisch in Form eines Granulats 5 eingefüllt.

Das Implantat ist dem gezeigten Beispiel ein Stent 1 mit einem rohrförmigen Grundkörper 2. Es versteht sich jedoch, dass die Erfindung auch im Zusammenhang mit beliebigen anderen Implantaten ihrer Anwendung finden kann. Es versteht sich weiterhin, dass hierbei beliebige Materialien für den Grundkörper des Implantats Anwendung finden können. Es ist lediglich erforderlich, dass dann das fließfähige oder schüttfähige Material bzw. Materialgemisch hierauf abgestimmt ist.

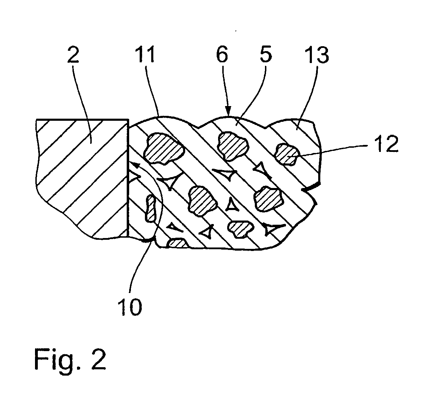

Das verfestigte Granulat 5 bildet ein Markerelement in Form eines Röntgenmarkers 6. Das Granulat 5 wird in einem Vorgang verfestigt, der einen endothermen Schritt umfasst. In diesem endothermen Schritt wird den Granulat 5 so viel Energie zugeführt, dass es zu einem Versintern der Körner des Granulats 5 kommt. Hierbei schmelzen die Körner des Granulats 5 an ihrer Oberfläche auf und verschmelzen so miteinander in den Bereichen, in denen sie aneinander anliegen. In einem weiteren Schritt kühlt das Material wieder ab und bildet dann auf Grund der sich ergebenden Verbindungen zwischen den Körnern des Granulat 5 einen Festkörper, der dann den Röntgenmarker 6 bildet. Dieser Zustand ist in Figur 2 dargestellt.

Um das Granulat 5 zur Ausbildung des Röntgenmarkers 6 zu verfestigen ist ein Laser 7 vorgesehen, mit dem das Granulat 5 gezielt bestrahlt wird, um die Prozessenergie für den endothermen Schritt in das Granulat 5 einzubringen. Der Laser strahlt dabei entlang seiner Strahlachse 8 auf das Granulat 5. Der Laser 7 kann in Richtung des Doppelpfeils 9 verfahren werden, um den gesamten Granulat 5 ausreichend Prozessenergie zuzuführen. Dabei wird die im Randbereich der Ausnehmung 3 eingebrachte Strahlungsenergiemenge so geregelt, dass es an der Grenzfläche 10 zwischen den Granulat 5 und dem Grundkörper 2 zusätzlich zu einem Aufschmelzen des Materials des Grundkörpers 2 kommt. Die Körner 11 des Granulats 5 verschmelzen somit oberflächlich mit dem Grundkörper 2, sodass sich eine Art Schweißverbindung zwischen dem Röntgenmarker 6 und dem Grundkörper 2 ergibt.

Das Granulat 5 besteht, wie Figur 2 zu entnehmen ist, aus einem Materialgemisch aus röntgenundurchlässigen Partikeln 12, die von einer Schicht 13 aus durch die Laserstrahlung aufschmelzbarem Material besteht. Es versteht sich jedoch, dass bei anderen bevorzugten Varianten an Stelle eines Materialgemisches auch zumindest oberflächlich aufschmelzbare Pulver- oder Granulatkörner aus einem einzigen, dann entsprechend strahlungsundurchlässigen Material Verwendung finden können.

Das Verfahren zum Anbringen des Röntgenmarkers 6 läuft wie folgt ab. Um das Einfüllen des Granulats 5 in die Ausnehmung 3 zu ermöglichen wird die zu beiden Seiten offene Ausnehmung 3 an ihrer Unterseite mit einem Verschlusselement 14 verschlossen. In die Ausnehmung 3 im Grundkörper 2 des Implantats 1 wird dann eine so große Menge des Granulats 5 eingefüllt, dass die Ausnehmung 3 annähernd vollständig verfüllt ist. Anschließend erfolgt der endotherme Schritt, in dem mittels des Lasers 7 den Granulat 5 sowie dem die Ausnehmung 3 begrenzenden Randbereich des Grundkörpers 2 in der oben beschriebenen Weise Energie zugeführt wird. Anschließend kühlt das versinterte Granulat 5 wieder ab und bildet so den fest mit dem Grundkörper 2 verbundenen Röntgenmarker 6.

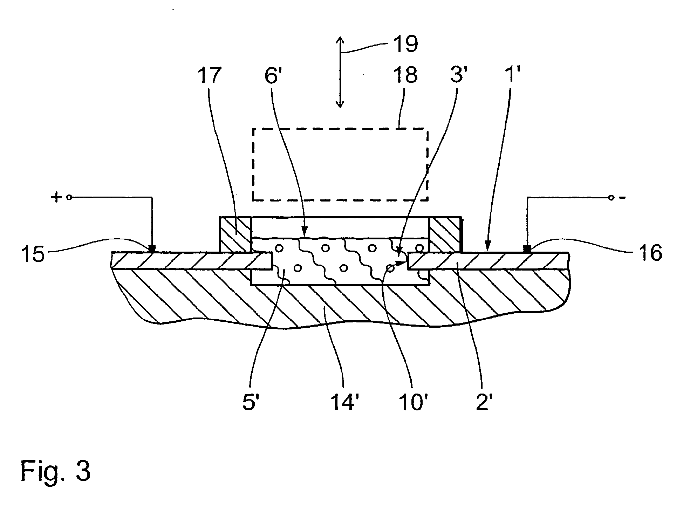

Figur 3 zeigt eine alternative Anordnung zur Durchführung einer Variante des erfindungsgemäßen Verfahrens. Diese gleicht in ihrem grundsätzlichen Ablauf derjenigen, die zu den Figuren 1 und 2 beschrieben wurde, sodass hier lediglich auf die Unterschiede eingegangen werden soll.

Ein Unterschied besteht in der Einbringung der Prozessenergie in dem endothermen Schritt des Verfahrens. Diese wird im gezeigten Beispiel über einen elektrischen Gleichstrom mittels der Kontakte 15 und 16 eingebracht, welche den elektrisch leitenden Grundkörper 2' des Implantats 1' kontaktieren. Die Kontakte 15 und 16 sind so angeordnet, dass der elektrische Gleichstrom durch das Granulat 5' fließt. Der elektrische Widerstand des Granulats 5', das im gezeigten Beispiel aus Körnern eines einzigen röntgenundurchlässigen Materials besteht, ist so groß, dass es infolge des Stromflusses im wesentlichen in der gesamten Granulatschüttung zu einer Erwärmung kommt, die ein Versintern der Körner des Granulats 5' in der zu Figur 1 und 2 beschriebenen Weise sicherstellt.

Ein zusätzlicher Unterschied besteht darin, dass die die Ausnehmung 3' begrenzenden Randbereiche 10' des Grundkörpers 2' einen besonders hohen elektrischen Widerstand aufweisen, um in diesem Bereich eine besonders große lokale Erwärmung zu erzielen, welche wiederum zu einem zu den Figuren 1 und 2 beschriebenen lokalen Verschmelzen der Körner des Granulats 5' mit dem Grundkörper 2' führt. Der hohe Widerstand wurde dabei durch eine Oxidation dieser Randbereiche 10' erzielt. Es versteht sich jedoch, dass er auch in beliebiger anderer Weise bewerkstelligt werden kann.

Ein weiterer Unterschied zur Variante aus Figur 1 besteht darin, dass eine zusätzliches Formelement 17 vorgesehen ist. Dieses ist zusätzlich zum Verschlusselement 14' im Bereich der oberen Öffnung der Ausnehmung 3' im Grundkörper 2' des Implantats 1' angeordnet. Das Verschlusselement 14' und das Formelement 17 sind dabei so ausgebildet und die Füllmenge des Granulats 5' ist so gewählt, dass sich Hinterschneidungen zwischen dem Grundkörper 2' und dem Röntgenmarker 6' ergeben. Mit anderen Worten ergibt sich ein Röntgenmarker 6', welcher den Randbereich der Ausnehmung 3' umgreift.

Um das Sintern des Granulats 5' zu unterstützen kann ein Stempel, wie er durch die gestrichelte Kontur 18 angedeutet ist, vorgesehen sein, der entlang des Pfeiles 19 verfahrbar ist. Mittels dieses Stempels kann das Granulat 5' beim Versintern mit Druck beaufschlagt werden, um das Sinterergebnis zu verbessern.

Es versteht sich, dass bei anderen Varianten des erfindungsgemäßen Verfahrens auch das Verschlusselement, das Formelement und/oder der Stempel einzeln oder in Kombination als Heizelemente ausgebildet sein können, um die Prozessenergie für den endothermen Schritt zur Verfügung zu stellen.

Es versteht sich weiterhin, dass das Verschlusselement 14' in dem gezeigten Beispiel entsprechend geringere Abmessungen als der Innenraum des Stents aufweist, sodass es nach Fertigstellung des Röntgenmarkers 6' radial von diesem entfernt und axial aus dem Stent herausgeführt werden kann.

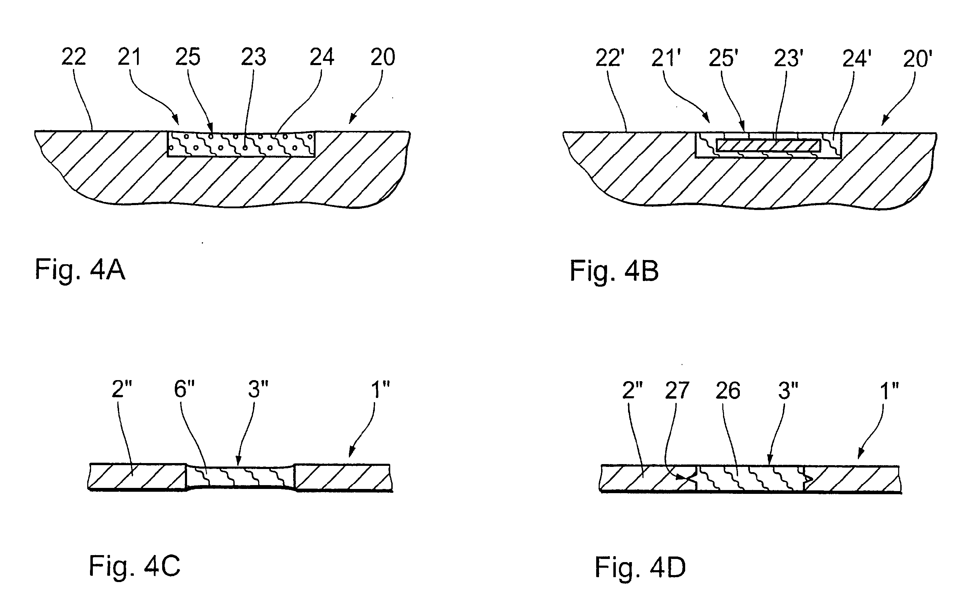

Figur 4A zeigt eine Variante eines erfindungsgemäßen Implantats 20 mit einer Ausnehmung 21 in Form einer Aushöhlung in einem Grundkörper 22. Die Ausnehmung 21 ist mit einer fließfähigen Suspension von röntgenundurchlässigen Partikeln 23 in einem aushärtenden Kunststoff 24 vergossen, die im ausgehärteten Zustand den Röntgenmarker 25 bildet.

Figur 4B zeigt eine weitere Variante eines erfindungsgemäßen Implantats 20' mit einer Ausnehmung 21' in Form einer Aushöhlung in einem Grundkörper 22'. Die Ausnehmung 21' ist mit einem fließfähigen, aushärtenden Kunststoff 24' vergossen, in dem wiederum einen röntgenundurchlässiges Element 23' eingebettet ist. Das Element 23' bildet zusammen mit dem ausgehärteten Kunststoffbett 24' den Röntgenmarker 25'.

Figur 4C zeigt eine weitere Variante eines erfindungsgemäßen Stents 1". Der Unterschied zu dem in Figur 1 dargestellten Stent besteht dabei darin, dass die Ausnehmung 3" im Grundkörper 2" in einem Tauchverfahren mit einer zähflüssigen Suspension verfülllt wurde, wie sie im Grundsatz zur Figur 4A beschrieben wurde. Die Verbindung zwischen dem sich ergebenden Röntgenmarker 6" und dem Grundkörper 2" ergibt sich dabei durch Adhäsion des Materials des Röntgenmarkers 6" an dem Grundkörper 2".

Figur 4D zeigt eine weitere Variante des erfindungsgemäßen Stents 1" mit einem Röntgenmarker 26. Der Unterschied zu dem in Figur 1 dargestellten Stent besteht zum einen darin, dass im die Ausnehmung 3" begrenzenden Bereich des Grundkörpers 2" eine oder mehrere Hinterschneidungen 27 vorgesehen sind, welche eine formschlüssige Verbindung zwischen dem Grundkörper 2" und dem Röntgenmarker 26 sicherstellen.

Ein weiterer Unterschied besteht darin, dass es sich bei dem für dem Röntgenmarker 26 verwendeten Material um aus der Zahnheilkunde bekanntes, kalt aushärtendes Amalgam handelt, welches hervorragende Röntgenopazität aufweist. Das Material wurde in seinem pastösen, nicht ausgehärteten Zustand in die Ausnehmung 3" eingebracht und konnte dort dann aushärten.

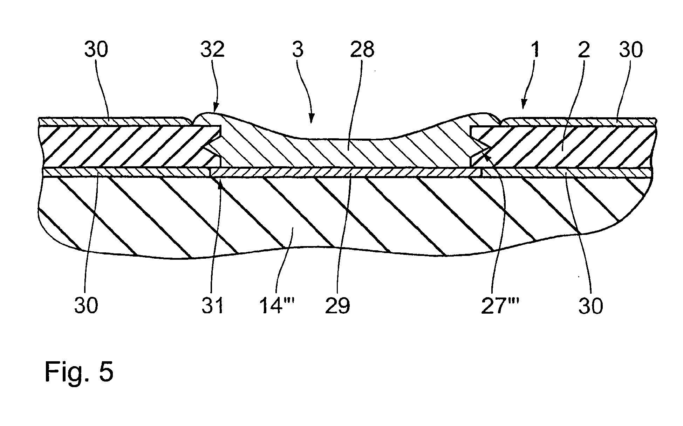

Figur 5 (nicht Teil der Erfindung) zeigt einen schematischen Teilschnitt durch eine weitere bevorzugte Ausführung des Implantats in Form eines Stents 1"' mit einem Grundkörper 2"', in dem sich eine Ausnehmung 3"' befindet. In diese Ausnehmung 3"' wurde durch galvanische Abscheidung eines röntgenopaken Materials, im vorliegenden Fall Gold, ein Röntgenmarker 28 eingebracht und verfestigt. Der Stent 1"' ist in Figur 5 in einem Zustand dargestellt, in dem die galvanische Abscheidung soeben abgeschlossen ist.

Zum Herstellen des Röntgenmarkers 8 wird der Stent 1"' in eine galvanische Lösung eingebracht, in der dann in bekannter Weise ein galvanischer Abscheidungsprozess abläuft. Der Grundkörper 2"' des Stents 1"' bildet dabei einem Teil der Ablagerungselektrode, an der sich das Material des Röntgenmarkers 28 anlagert. Um sicherzustellen, dass die Ausnehmung 3"' in der dargestellten Weise ausgefüllt wird, ist ein weiterer Teil der Ablagerungselektrode von einer dünnen, leitenden Beschichtung 29 auf dem im Innern des Stents 1"' angeordneten Verschlusselement 14"' gebildet, welche den die Ausnehmung 3"' begrenzenden Randbereich des Grundkörpers 2"' leitend kontaktiert.

Es versteht sich, dass je nach Geometrie der Ausnehmung gegebenenfalls auch auf eine solche leitende Beschichtung oder dergleichen zum Ausfüllen der Ausnehmung verzichtet werden kann. Vielmehr ist es möglich, dass die Ausnehmung auch ohne ein solches Hilfsmittel im Zuge der galvanischen Abscheidung "zuwächst".

Um zu verhindern, dass sich das Material für den Röntgenmarker 28 an dem gesamten Grundkörper 2"' des Stents 1"' anlagert, ist dieser in den Bereichen, in denen keine Anlagerung stattfinden soll, mit einer leicht entfernbaren Schutzbeschichtung 30 versehen, die eine solche Anlagerung verhindert. Hierbei kann es sich beispielsweise um eine Wachs- oder Fettschicht oder dergleichen handeln.

Beim späteren Lösen des Verschlusselements 14"' aus dem Stent bleibt die Beschichtung 29 an dem Röntgenmarker 28 haften und löst sich vom Verschlusselement 14"'. Dies ist besonders dann sinnvoll, wenn die Beschichtung ohnehin aus demselben Material wie der Röntgenmarker 28 besteht, da dann eine besonders gute Verbindung sichergestellt ist. Zum anderen ergibt sich mit der gezeigten Konfiguration eine Überlappung 31 zwischen Röntgenmarker 28 und Grundkörper 2"', welche zusammen mit der Überlappung 32 zwischen Röntgenmarker 28 und Grundkörper 2"' zur festen mechanischen Verbindung zwischen Röntgenmarker 28 und Grundkörper 2"' beiträgt.

Eine weitere derartige mechanische Fixierung ergibt sich durch die Hinterschneidung 27"'. Es versteht sich jedoch, dass diese nicht notwendigerweise vorgesehen sein muss. Ebenso kann diese die beschriebenen Überlappungen ersetzen. Weiterhin kann auch die Haftkraft des Röntgenmarkermaterials an dem Material des Grundkörpers groß genug sein, um eine ausreichende Fixierung des Röntgenmarkers sicherzustellen.

Es versteht sich im übrigen, dass sich die einen Teil der Ablagerungselektrode bildende, elektrisch leitende Beschichtung bei anderen Varianten der Erfindung beim Entfernen des Verschlusselements auch von dem Röntgenmarker lösen kann, sodass der Röntgenmarker dann nur aus dem galvanisch abgeschiedenen Material - gegebenenfalls mit den beschriebenen Einlagerungen etc. - besteht.

Weiterhin versteht es sich, dass wie schon oben insbesondere zu Figur 4B beschrieben in dem galvanisch abgeschiedenen Material auch Partikel oder Teile aus einem anderen oder gegebenenfalls sogar aus demselben Material eingebettet sein können.

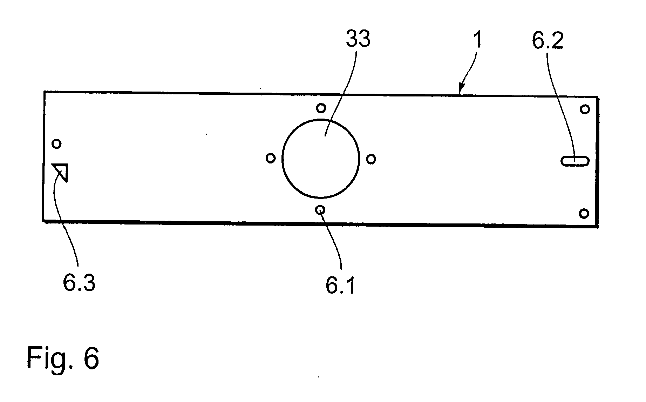

Figur 6 zeigt eine schematische Draufsicht auf den Stent 1 aus den Figuren 1 und 2. Bei diesem handelt es sich um einen so genannten Bifurkationsstent, der im Bereich von Gefäßverzweigungen eingesetzt wird. Hierzu weist er eine seitliche Öffnung 33 auf, die durch Röntgenmarker 6.1 gekennzeichnet ist.

An seinen beiden Enden weist der Stent 1 weitere Röntgenmarker 6.2 und 6.3 auf. Diese dienen zum einen dazu, die Enden des Stents 1 zu markieren. Zum anderen ist ihre Gestalt sowie die Anzahl der Röntgenmarker 6.2 bzw. 6.3 so gewählt, dass sie dem Chirurgen unterschiedliche Informationen bezüglich des Stents liefern. So geben Form und Anzahl der Röntgenmarker 6.2 Aufschluss über das Herstellungsdatum des Stents 1, während Form und Anzahl der Röntgenmarker 6.3 eine Produktkennung für den Stent darstellen. Es versteht sich, dass auch beliebige andere Informationen bezüglich des Stents durch Form, Anzahl und Anordnung der Röntgenmarker wiedergegeben werden können.

Es versteht sich weiterhin, dass die erfindungsgemäßen Markerelemente nicht nur in gesondert dafür vorgesehene Ausnehmungen in der Stentwandung eingebracht werden können. Ebenso können Sie in vorteilhafter Weise durch die Netzstruktur der Stentwandung vorgegebene Ausnehmungen eingebracht werden. Hierbei können sie beliebig über den Stent verteilt werden.

## Patentansprüche

1. Verfahren zum Anbringen eines Markerelements (6; 6'; 6"; 25; 25'; 26; 28) an einem zum Implantieren in den menschlichen oder tierischen Körper vorgesehenen Implantat (1; 1'; 1''; 1'''; 20; 20'), insbesondere einem Stent, mit einem Grundkörper und einer in diesem Grundkörper (2; 2'; 2"; 2'''; 22; 22') vorgesehenen Ausnehmung (3; 3'; 3"; 3'''; 21; 21') zur Aufnahme des Markerelements (6; 6'; 6"; 25; 25'; 26; 28), wobei zur Ausbildung des Markerelements (6; 6'; 6"; 25; 25'; 26; 28) ein verfestigbares Material In die Ausnehmung (3; 3'; 3"; 3'''; 21; 21') eingebracht und dort verfestigt wird, **dadurch gekennzeichnet, dass** das verfestigbare Material ein Materialgemisch ist bestehend aus Im wesentlichen strahlungsundurchlässigen Partikeln oder größeren Teilen und einer diese strahlungsundurchlässigen Partikel oder größeren Teile umschließenden oder umgebenden verfestigbaren Komponente.

2. Verfahren nach Anspruch 1, **dadurch gekennzeichnet, dass** das verfestigbare Materialgemisch aus der Gruppe umfassend Kunststoffe, Kunststoffgemische, Metalle, Metalliegierungen und Keramiken auswählbar ist.

3. Verfahren nach Anspruch 1 oder 2, **dadurch gekennzeichnet, dass** zur Ausbildung des Markerelements (6; 6'; 6"; 25; 25') ein verfestigbares, fließ- oder schüttfähiges Materialgemisch In die Ausnehmung eingebracht und dort verfestigt wird.

4. Verfahren nach Anspruch 3, **dadurch gekennzeichnet, dass** das fließ- oder schüttfähige Materialgemisch ein sinterfähiges Granulat oder Pulver ist, das in der Ausnehmung durch Sintern verfestigt wird.

5. Verfahren nach Anspruch 3 oder 4, **dadurch gekennzeichnet, dass** das fließ- oder schüttfähige Materialgemisch während des Verfestigungsvorgangs mit dem Material des Grundkörpers (2; 2'; 2"; 22; 22') verbunden, insbesondere verschweißt wird.

6. Verfahren nach einem der vorhergehenden Ansprüche, **dadurch gekennzeichnet, dass** der Verfestigungsvorgang einen endothermen Schritt umfasst und zumindest ein Teil der Prozessenergie in dem endothermen Schritt lokal Im Bereich der Ausnehmung eingebracht wird.

7. Verfahren nach Anspruch 6, **dadurch gekennzeichnet, dass** zumindest ein Teil der Prozessenergie In dem endothermen Schritt durch gezielte Bestrahlung im Bereich der Ausnehmung, insbesondere mit Laserstrahlung, eingebracht wird.

8. Verfahren nach Anspruch 6 oder 7, **dadurch gekennzeichnet, dass** zumindest ein Teil der Prozessenergie in den endothermen Schritt durch Ultraschall eingebracht wird.

9. Verfahren nach einem der vorhergehenden Ansprüche, **dadurch gekennzeichnet, dass** der Verfestigungsvorgang einen endothermen Schritt umfasst und zumindest ein Teil der Prozessenergie in dem endothermen Schritt elektrisch durch Erzeugung eines Stromflusses durch das Im Bereich der Ausnehmung (3; 3'; 3"; 21; 21') angeordnete fließ- oder schüttfähige Materialgemisch eingebracht wird.

10. Verfahren nach Anspruch 1 oder 2, **dadurch gekennzeichnet, dass** das Einbringen und Verfestigen des Materialgemisches durch galvanische Abscheidung erfolgt.

11. Verfahren nach Anspruch 1 oder 2, **dadurch gekennzeichnet, dass** ein kalt aushärtendes Materialgemisch, insbesondere Amalgam verwendet wird.

12. Implantat, insbesondere Stent, zum Implantieren in den menschlichen oder tierischen Körper mit einem Grundkörper (2; 2'; 2"; 2"'; 22; 22'), wenigstens einer Ausnehmung (3; 3'; 3"; 3"'; 21; 21') in diesem Grundkörper (2; 2'; 2"; 2"'; 22; 22') und einem in dieser Ausnehmung (3; 3'; 3"; 3"'; 21; 21') angeordneten Markerelement (6; 6'; 6"; 25; 25'; 26; 28) aus verfestigbarem Material, welches in die Ausnehmung eingebracht und dort verfestigt ist, **dadurch gekennzeichnet, dass** das verfestigbare Material ein Materialgemisch ist bestehend aus im wesentlichen strahlungsundurchlässigen Partikeln oder größeren Tellen und einer diese strahlungsundurchlässigen Partikel oder größeren Teile umschließenden oder umgebenden verfestigbaren Komponente.

13. Implantat nach Anspruch 12, **dadurch gekennzeichnet, dass** die Ausnehmung (3; 3'; 3"; 21; 21') und/oder das Markerelement (6; 6'; 6"; 25; 25') und/oder deren Anordnung bezüglich des Grundkörpers (2; 2'; 2"; 22; 22') zur Kennzeichnung wenigstens einer Eigenschaft des Implantats ausgebildet sind.

## Claims

1. A method for fitting a marker element (6; 6'; 6"; 25; 25'; 26; 28) to an implant (1; 1'; 1"; 1"'; 20; 20') provided for implanting in the human or animal body, in particular a stent, with a base body and a recess (3; 3'; 3"; 3"'; 21; 21') provided in this base body (2;, 2'; 2"'; 22; 22') for receiving the marker element (6; 6', 6"; 25; 25', 26; 28), wherein a hardenable material is inserted in the recess (3; 3'; 3"; 3"'; 21; 21') for forming the marker element (6; 6'; 6"; 25; 25', 26; 28) and is hardened there, **characterised in that** the hardenable material is a material mixture consisting of essentially radiation-impermeable particles or larger parts and a hardenable component enclosing or surrounding these radiation-impermeable particles or larger parts.

2. The method according to Claim 1, **characterised in that** the hardenable material mixture may be selected from the group comprising plastics, plastic mixtures, metals, metal alloys and ceramics.

3. The method according to Claim 1 or 2, **characterised in that** a hardenable, flowable or pourable material mixture is inserted in the recess and hardened there to form the marker element (6; 6'; 6"; 25; 25').

4. The method according to Claim 3, **characterised in that** the flowable or pourable material mixture is a sinterable granulate or powder, which is hardened in the recess by sintering.

5. The method according to Claim 3 or 4, **characterised in that** the flowable or pourable material mixture during the hardening process is connected, in particular welded, to the material of the base body (2; 2'; 2"; 22; 22') during the hardening process.

6. The method according to one of the preceding claims, **characterised in that** the hardening process comprises an endothermic step and **in that** at least some of the process energy is introduced in the endothermic step locally in the region of the recess.

7. The method according to Claim 6, **characterised in that** at least some of the process energy is introduced in the endothermic step by specific irradiation in the region of the recess, in particular with laser radiation.

8. The method according to Claim 6 or 7, **characterised in that** at least some of the process energy is introduced in the endothermic step by ultrasound.

9. The method according to one of the preceding claims, **characterised in that** the hardening process comprises an endothermic step and **in that** at least some of the process energy is introduced electrically in the endothermic step by generating a flow of current through the flowable or pourable material mixture arranged in the region of the recess (3; 3'; 3"; 21; 21').

10. The method according to Claim 1 or 2, **characterised in that** the material mixture is introduced and hardened by galvanic precipitation.

11. The method according to Claim 1 or 2, **characterised in that** a cold hardening material mixture, in particular amalgam, is used.

12. An implant, particularly a stent, for implanting in the human or animal body with a base body (2; 2'; 2"; 2"'; 22; 22'), at least one recess (3; 3', 3"; 3"'; 21; 21') in this base body (2; 2'; 2"; 2"'; 22; 22') and a marker element (6; 6'; 6"; 25; 25'; 26; 28) arranged in this recess (3; 3'; 3"; 3"'; 21; 21') of hardenable material which is inserted in this recess and hardened there, **characterised in that** the hardenable material is a material mixture consisting of essentially radiation-impermeable particles or larger parts, and a hardenable component enclosing these radiation-impermeable particles or larger parts.

13. The implant according to Claim 12, **characterised in that** the recess (3; 3', 3"; 21; 21') and/or the marker element (6; 6'; 6"; 25; 25') and/or its arrangement relative to the base body (2; 2'; 2"; 22; 22') are formed for identifying at least one characteristic of the implant.

## Revendications

1. Procédé pour placer un élément marqueur (6, 6' ; 6" ; 25 ; 25' ; 26 ; 28) sur un implant (1 ; 1' ; 1" ; 1"' ; 20 ; 20') prédéfini pour l'implantation dans le corps humain ou animal, en particulier un stent, comprenant un corps de base et un évidement (3 ; 3' ; 3" ; 3"' ; 21 ; 21') prévu dans ce corps de base (2 ; 2' ; 2" ; 2"' ; 22 ; 22') pour le logement de l'élément marqueur (6, 6' ; 6" ; 25 ; 25' ; 26; 28), un matériau durcissable étant introduit dans l'évidement (3; 3' ; 3" ; 3"' ; 21 ; 21') pour la réalisation de l'élément marqueur (6, 6' ; 6" ; 25 ; 25' ; 26 ; 28) et étant fixé à cet endroit, **caractérisé en ce que** le matériau durcissable est un mélange de matériau constitué de particules sensiblement imperméables au rayonnement ou de parties plus grandes et d'un composant durcissable entourant ou enveloppant ces particules imperméables au rayonnement ou parties plus grandes.

2. Procédé selon la revendication 1, **caractérisé en ce que** le mélange de matériau durcissable peut être sélectionné parmi les groupes comprenant des plastiques, des mélanges de plastique, des métaux, des alliages de métaux et des céramiques.

3. Procédé selon la revendication 1 ou 2, **caractérisé en ce que**, pour la réalisation de l'élément marqueur (6 ; 6' ; 6" ; 25 ; 25'), un mélange de matériau durcissable, fluide ou déversable est introduit dans l'évidement et y est fixé.

4. Procédé selon la revendication 3, **caractérisé en ce que** le mélange de matériau coulant ou déversable est un granulat ou une poudre frittable, qui est durci dans l'évidement par frittage.

5. Procédé selon la revendication 3 ou 4, **caractérisé en ce que** le mélange de matériau coulant ou déversable est relié, en particulier soudé, pendant l'opération de durcissement avec le matériau du corps de base (2 ; 2' ; 2" ; 22 ; 22').

6. Procédé selon l'une quelconque des revendications précédentes, **caractérisé en ce que** l'opération de durcissement comprend une étape endotherme et au moins une partie de l'énergie de processus est introduite dans l'étape endotherme localement dans la zone de l'évidement.

7. Procédé selon la revendication 6, **caractérisé en ce qu'**au moins une partie de l'énergie de processus est introduite lors de l'étape endotherme par une irradiation ciblée dans la zone de l'évidement, en particulier avec du rayonnement laser.

8. Procédé selon la revendication 6 ou 7, **caractérisé en ce qu'**au moins une partie de l'énergie de processus est introduite dans l'étape endotherme par des ultrasons.

9. Procédé selon l'une quelconque des revendications précédentes, **caractérisé en ce que** l'opération de durcissement comprend une étape endotherme et au moins une partie de l'énergie de processus est introduite lors de l'étape endotherme électriquement par génération d'un flux de courant à travers le mélange de matériau coulant ou déversable dans la zone de l'évidement (3 ; 3' ; 3" ; 21; 21').

10. Procédé selon la revendication 1 ou 2, **caractérisé en ce que** l'introduction et le durcissement du mélange de matériau s'effectue par précipitation galvanique.

11. Procédé selon la revendication 1 ou 2, **caractérisé en ce qu'**un mélange de matériau durcissant à froid, en particulier de l'amalgame, est utilisé.

12. Implant, en particulier stent, pour l'implantation dans le corps humain ou animal avec un corps de base (2 ; 2' ; 2" ; 2''' ; 22 ; 22'), au moins un évidement (3 ; 3' ; 3" ; 3"' ; 21 ; 21') dans ce corps de base (2 ; 2' ; 2" ; 2''' ; 22 ; 22') et un élément marqueur (6 ; 6' ; 6" ; 25 ; 25' ; 26 ; 28) disposé dans cet évidement (3 ; 3' ; 3" ; 3''' ; 21 ; 21') à base de matériau durcissable, qui est introduit dans l'évidement et y est fixé, **caractérisé en ce que** le matériau durcissable est un mélange de matériau constitué de particules sensiblement imperméables au rayonnement ou de parties assez grandes et d'un composant durcissable entourant ou enveloppant ces particules imperméables au rayonnement ou parties plus grandes.

13. Implant selon la revendication 12, **caractérisé en ce que** l'évidement (3 ; 3' ; 3" ; 21 ; 21') et/ou l'élément marqueur (6 ; 6' ; 6" ; 25 ; 25') et/ou son agencement par rapport au corps de base (2 ; 2' ; 2" ; 22 ; 22') sont réalisés pour l'identification d'au moins une propriété de l'implant.
